# EUROPEAN PATENT SPECIFICATION

(11) **EP 0 784 699 B1**
(45) Date of publication and mention of the grant of the patent: **14.07.1999**
(21) Application number: 95930671.3
(22) Date of filing: 31.08.1995
(51) Int. Cl.: C12Q 1/00, C12Q 1/18, G01N 33/483

(54) **ASSAY AND REAGENT KIT FOR THE QUANTITATIVE IN VITRO DETERMINATION IN BIOLOGICAL SPECIMENS OF THE ACTIVITY OF PROTEINS CAUSING MULTI-DRUG RESISTANCE IN TUMORS**
TEST- UND REAGENZIENSATZ FÜR DIE QUANTITATIVE IN VITRO BESTIMMUNG DER AKTIVITÄT VON PROTEINEN, DIE MEHRFACHRESISTENZ GEGEN CHEMOTHERAPEUTIKA IN TUMOREN VERURSACHEN, IN BIOLOGISCHEN PROBEN
TITRAGE ET TROUSSE DE REACTIFS PERMETTANT DE QUANTIFIER IN VITRO DANS DES SPECIMENS BIOLOGIQUES L'ACTIVITE DE PROTEINES RESPONSABLES DE LA RESISTANCE MULTIPLE AUX ANTICANCEREUX

(30) Priority: 31.08.1994 HU 9402511
(43) Date of publication of application: 23.07.1997
(73) Proprietor: Sarkadi, Balazs, 1121 Budapest (HU); Homolya, Laszlo, 1023 Budapest (HU); Hollo, Zsolt, 1119 Budapest (HU)
(72) Inventor: Sarkadi, Balazs, 1121 Budapest (HU); Homolya, Laszlo, 1023 Budapest (HU); Hollo, Zsolt, 1119 Budapest (HU)
(74) Representative: Pett, Christopher Phineas
(86) International application number: PCT/HU95/00042
(87) International publication number: WO 96/06945

(56) References cited:
- US-A- 5 403 574
- CHEMICAL ABSTRACTS, Vol. 123, No. 17, 23 October 1995, (Columbus, Ohio, USA), page 14, Abstract No. 217629e, C.P. LEITH et al., "Correlation of Multidrug Resistance (MDR1) Protein Expression with Functional Dye/Drug Efflux in Acute Myeloid Leukemia by Multiparameter Flow Cytometry: Identification of Discordant MDR-/Efflux+ and MDR1+/Efflux-Cases"; & BLOOD, 1995, 86(6), 2329-42.

## Description

The present invention relates to a quantitative *in vitro* diagnostic assay method for the determination of the activity of transport proteins causing multi-drug resistance in tumors (MDR proteins) and for the determination of the concentration of the biologically active MDR protein in the specimen. The invention further relates to reagent kits for performing said assay method.

The method according to the invention comprises contacting the biological specimen - or a part of said specimen - with a derivative of an easily detectable compound, said derivative being a good substrate of the MDR protein present in the specimen and being capable of entering the cells of the specimen where said derivative is transformed to said easily detectable compound as a consequence of the activity of an enzyme or enzymes, said easily detectable compound not being a substrate (or not being a good substrate) of said MDR protein and consequently said easily detectable compound accumulates within the cells. The method of the invention further comprises the quantitative determination of the accumulation rate of said easily detectable compound within the cells by the means of measuring a detectable signal (e.g. fluorescence or light absorption); and contacting the biological specimen - or another part of said specimen - with a derivative of said easily detectable compound in the presence of a transport blocking agent; quantitatively determining the accumulation rate of said easily detectable compound within the cells by the same manner; and calculating - and expressing in desired diagnostic units - the activity and/or concentration of said biologically active MDR protein present in said specimen by applying an empirically established correlation.

The method and reagent kits according to the invention are especially useful in human clinical diagnostics for making a fast and simple estimation on the measure of the multi-drug resistance of tumors based on the measurement of their MDR activity which is of particular help in establishing the proper chemotherapeutic treatment required.

With respect to the present specification, we will use the foregoing technical terms in accordance with the given definition. With regard to the interpretation of the present invention, it shall be understood that the below defined terms are used in accordance with the given definitions even if said definitions are not in perfect hannony with the usual interpretation of said technical term.

A *"transport protein"* is a membrane bound protein which can transport a given compound through the cell membrane even against the concentration gradient by using the energy obtained through ATP hydrolysis.

A *"transport blocking agent"* is a compound that can block the active transport of a compound of interest (e.g. calcein-AM) through the cell membrane by any mechanism of action. Transport blocking agent can be, for example, an inhibitor of the transport protein or a good substrate of same applied in excess of the amount of the compound of interest present.

*"MDR1 or P-glycoprotein*" is a membrane bound protein of 170 kD molecular weight, functional expression of which causes multi-drug resistance of the given cell.

With respect to the present invention the gene product of the MDR1 gene together with its functional analogues are named *"MDR proteins"* without any regard to their source or to their usually accepted trivial names (for example, the protein called MRP in the literature which is homologous to MDR1 will also be termed an MDR protein here). In other words: the term *"MDR protein"* stands in the present specification for every protein causing multi-drug resistance.

A *"substrate of an MDR protein"* is a compound that can be extruded from the cell through an MDR mediated active transport mechanism (e.g. cytotoxic agents, cytostatic agents and other foreign extracellular compounds). *"Good"* substrates of an MDR protein are extruded significantly faster from the cell than those termed *"not good"* substrates.

The term *"multi-drug resistance"* denotes a drug resistance of wide spectrum caused by the functional expression of an MDR protein. When, for example, a tissue becomes multi-drug resistant, it will be resistant for a wide variety cytotoxic and cytostatic agents of significantly different structure.

Every protein that has a function identical or similar to another protein will be termed here a *"functional analogue"* of said second protein. Functional analogues of a protein are also structural homologues of said protein in most cases. For example, every membrane transport protein that can extrude compounds from a cell which are detrimental to any function of said cell through an active transport mechanism will be termed functional analogues of the MDR1 protein.

*"Structurally homologous proteins"* are those proteins that share at least one important domain or other important part of similar tertiary structure.

The term "*MDR activity"* denotes the activity of a protein causing multi-drug resistance, i.e. the activity of the MDR1 protein and its functional analogues.

An *"easily detectable compound"* is a compound the presence and/or the concentration of which can be accurately determined by eye sight or by instrumental measurement under the given conditions based on a detectable characteristic of said compound. Examples of such compounds are: colored compounds, fluorescent compounds, luminescent compounds and compounds having special catalytic or enzyme activity or compounds necessary for such activities (e.g. coenzymes).

The ATP hydrolyzing capability of an MDR protein, through which it provides energy for the active membrane transport, is termed *"MDR dependent ATPase"* activity herein.

"*Cell permeable compound*" is a usually hydrophobic compound which can enter the cell in a practically unhindered manner.

The efficiency of chemotherapy of tumors is seriously affected or hindered by the onset of the so called multi-drug resistance that, with respect to human tumors, is caused by the expression of a membrane bound protein of 170,000 derivative molecular weight called MDRI protein alternatively termed P-glycoprotein or its functional analogues in the malignant tissue. The MDR proteins extrude the applied cytostatic agents (e.g. Vinca alkaloids, anthracycline derivatives and other clinically used effective anti cancer agents) by active membrane transport making use of the energy obtained from ATP hydrolysis thus making practically impossible for said agents to produce their cytostatic effect [Gottesman, M. M. et al, Annu. Rev. Biochem. 62, 385 (1993); Arceci, R. J., Blood 81, 2215 (1993)].

There are a number different methods employed for the clinical examination of the MDR proteins and for the detection of said protein in tumor cells, the reliability of said methods is presently questionable. It is particularly difficult to elaborate a functional *in vitro* assay method really characterizing the expectable spectrum of the drug resistance caused by the MDR proteins. Presently mainly cytotoxicity assays [Bruggemann, E. P. et al, J. Biol. Chem. 267, 21020 (1992)] and measurement methods for the extrusion of drug simulating dyes [Goldstein, L. J. et al, Oncology/Haematology 12, 243 (1992)] are used for such purposes. It has been shown that the accumulation in MDR protein expressing tissues of certain fluorescent anthracycline derivatives [Herweijer, H. et al, Cytometry 10, 463 (1989)] and fluorescent dyes such as rhodamine 123 [Neyfakh, A. A., Exp. Cell. Res. 174, 168 (1988)] and fluo-3 [Wall, D. M. et al, J. Natl. Cancer Inst. 83, 206 (1991)] is reduced, therefore these compounds are now used for the detection of the presence of MDR proteins. The reliability of these methods, however, are significantly affected by numerous principal and technical problems, such as change in fluorescence emission upon the binding of the applied dye within the cell, accumulation of the applied agent in different cell organelles, metabolism of said agents, change in pH and in concentration of different ions, and leakage of the agents through the cell membrane [Gottesman, M. M. et al, Annu. Rev. Biochem. 62, 385 (1993); Weaver, J. L. et al, Exp. Cell. Res. 196, 323 (1991)]. It is known, for example, that when applying fluo-3-AM (i.e. fluo-3-acetoxy-methylester) in such a method, the calcium dependent fluorescence of the free dye interferes with the evaluation of the assay [Handbook of fluorescent probes and research chemicals, pp. 115-116, ed. Haughland, R. P., Molecular Probes Inc., Eugene, OR (1992-94)].

The elaboration of the present invention was directly preceded by the observation of the present inventors that the hydrophobic cell permeable ester derivatives of some fluorescent dyes are actively extruded from the cells by MDR proteins present is said cells, but said MDR proteins can not extrude the free dye compounds produced from said derivatives by esterases present in the cell [Homolya, L. et al, J. Biol. Chem., 268, 21493 (1993)]. It has been demonstrated that the commercially available compound, calcein-AM (calcein-acetoxy-methylester), generally used in cell viability assays, is - unlike free calcein - an excellent activator of the MDR dependent ATPase (Kₐ ≤1 µmol/l). It has also been shown that calcein accumulation in the cell following calcein-AM uptake is reduced by the presence of MDR activity.

Calcein-AM is practically non fluorescent and highly lipid soluble (hydrophobic) thus rapidly penetrates through the cell membrane. Intracellular esterases rapidly cleave the ester bond present in the compound thus producing a highly fluorescent water soluble (hydrophilic) compound(s). When living cells are contacted with calcein-AM, calcein-AM molecules continuously penetrate the cells because of the established concentration gradient and cleavage products accumulate within the cell. The usage of calcein-AM in cell viability assays is also based on this principle since in damaged or dead cells entering calcein-AM molecules will not be transformed to a fluorescent product [Handbook of fluorescent probes and research chemicals, pp. 172-173, ed. Haughland, R. P., Molecular Probes Inc., Eugene, OR (1992-94)]. Application of calcein for studying of liposome fusion and lysis is also known in the art [Kendall, D. A. et al, Anal. Biochem. 134, 26 (1983); Allen, T. M., Liposome Technol. 3, 177 (1984)].

In their latest article published in the subject matter [Holló, Zs. et al, Biochim. Biophys. Acta 1191, 384 (1994)], the present inventors have demonstrated that calcein-AM is useful for the qualitative functional analysis of the presence of MDRI activity. If MDR1 activity is present in the cell membrane, it extrudes the penetrating calcein-AM molecules from the cells via an active transport mechanism and thus the rate of transformation of calcein-AM to fluorescent calcein (or other fluorescent calcein derivatives) and of accumulation of the fluorescent product(s) within the cells will be significantly reduced.

As calcein is a derivative of fluorescein, its molar fluorescence emission coefficient is very high and its excitation and emission parameters (ex.: 490 nm; em.: 515 nm) make calcein a very good candidate for application in flow cytometric measurements, in conventional fluorescence detection systems or even in scanning laser microscopy. The free calcein molecules produced within the cells do not leak out from the cells, are not detrimental to the cells, and their fluorescent characteristics are highly independent from different influences, thus the fluorescence of calcein is practically resistant to changes in the pH or in the concentration of Ca²⁺ or Mg²⁺ ions and no component usually present in the cells interferes with said fluorescence.

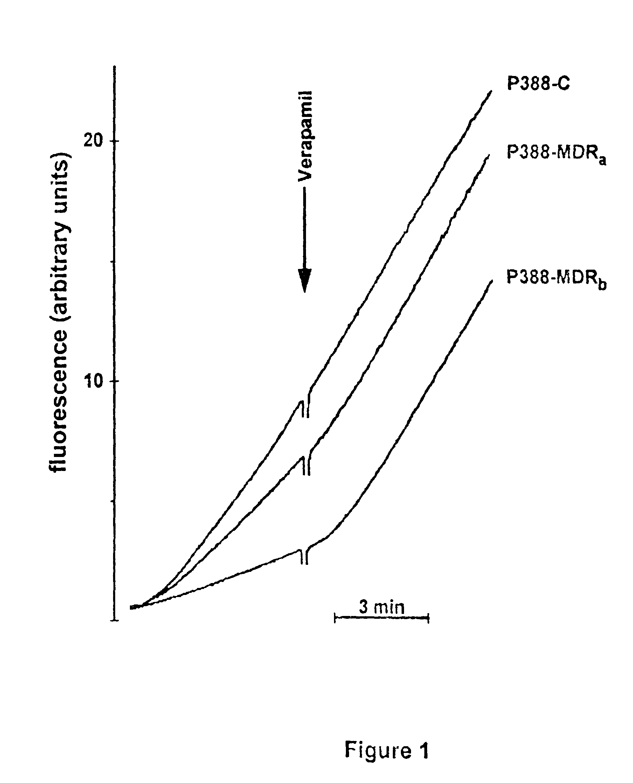

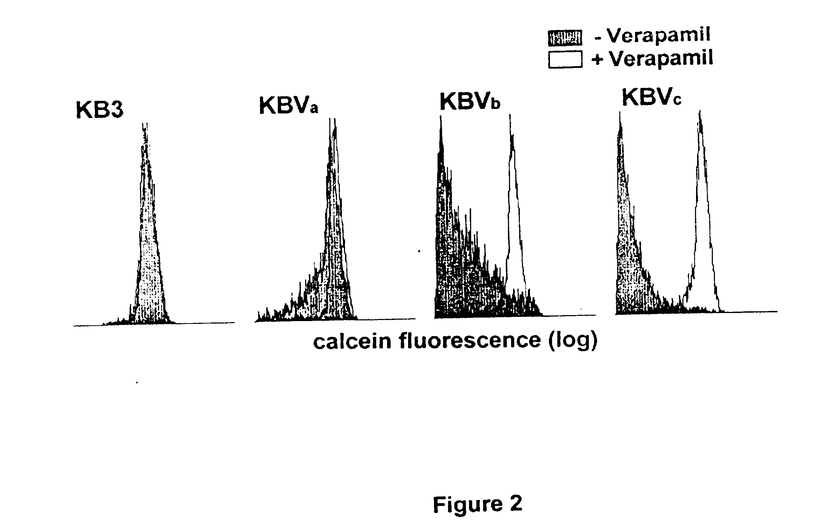

As it was previously said, when contacting cells with calcein-AM accumulation of calcein (derivatives) in cells where MDR activity is present is very slow compared to cells lacking MDR activity or having a substantially reduced MDR activity. Good substrates of the MDR proteins (when applying them in excess) and their inhibitors such as verapamil, vinblastin, cyclosporin A and oligomycin [Gottesman, M. M. et al, Annu. Rev. Biochem. 62, 385 (1993); Arceci, R. J., Blood 81, 2215 (1993)] are blocking the calcein-AM transporting activity of the MDR protein, thus upon addition of such agents to MDR protein containing cells contacted with calcein-AM, enhancement of fluorescence emission within the cells significantly accelerates [Homolya, L. et al, J. Biol. Chem., 268, 21493 (1993)]. Under similar conditions, addition of verapamil or vinblastin to cells not containing MDR proteins has no effect on the rate of accumulation of the fluorescent dye. Thus one can compare the rate of fluorescence enhancement in the same cell types carrying out parallel measurements with and without applying a blocking agent of the MDR dependent active transport. For cells lacking MDR activity the two measured values will be the same but for cells expressing MDR protein the two values will differ significantly (see Ex. 1, Fig. 1 and 2).

Considering the above summarized state of the art one can easily conclude that elaboration of a quantitative *in vitro* clinical diagnostic assay method making possible, by applying a relatively simple and inexpensive measurement, the reliable pre-estimation of the measure of multi-drug resistance of different tumor types would be of high economic and pharmaceutical significance.

It should be contemplated that for solving this above defined task, it is not enough to only detect the presence of MDR activity qualitatively in the assayed malignant tissue, since the knowledge of the accurate measure of the developed multi-drug resistance is crucial for the establishment of the proper chemotherapy to be employed. As a chemotherapeutic treatment of a cancer patient presently costs thousands of US dollars, it should be understood that the availability of a reliable quantitative diagnostic assay method for the determination of the MDR protein expressed in the tumor would be of high economic significance as by applying such a method one could accurately estimate the effective amount of the chemotherapeutic agents to be applied or alternatively, it could be pre-determined whether the given tumor will at all respond to the cytotoxic agent planned to be employed or not. There is another expected therapeutic advantage of the quantitative *in vitro* diagnostic assay method of the invention that is also of significance. With the help of this method the minimal effective amount of the cytotoxic agent to be employed can be calculated thus one can significantly reduce the detrimental side effects of chemotherapy. The method of the invention is also suitable for the large scale screening and testing of multi-drug resistance reversing agents produced by the pharmaceutical industry.

It is, therefore, necessary to elaborate an assay method by which it is possible to obtain a diagnostically useful value reliably indicating the measure of the MDR activity of the specific tumor type in question. Presently there is no such quantitative MDR diagnostic method available for physicians and the presently applied qualitative MDR detecting research methods are not very likely to get developed to become easily performable quantitative diagnostic assay methods because of the problems and difficulties described above.

The present invention is based on the recognition that it is possible to elaborate a simple assay method quantitatively characterizing the concentration of the biologically active MDR transport protein in cells, i.e. the maximum MDR activity of the specimen. Such method comprises the following steps: contacting tumor cells *in vitro* with calcein-AM; measuring the rate of accumulation of fluorescent calcein derivative(s) within the cell; and calculating an MDR activity factor (MAF) from the measured value using an internal standard by applying empirically established correlations, said MAF being a dimensionless number and being characteristic of the measure of the MDR activity of the assayed cells regardless of the type, size and origin of the assayed cells. For calculating MAF, the measure of calcein accumulation in the cells of the assayed specimen determined in the presence of a transport blocking agent is used as the internal standard. It has been demonstrated that the concentration of MDR protein in different multi-drug resistant tumor cell lines deduced from the MAF calculated according to the method of the invention is - within the clinically important concentration range (0-0.2 µg MDR protein / mg total protein) - in very good correlation with the concentration of the functional MDR protein measured by immunological methods in the same cell lines (see Ex. 1 and Fig. 1).

It is important to emphasize with respect to the clinical diagnostic applicability of the method according to the invention that MAF calculated according to the process of the invention was - in all cases examined so far - in good correlation with the really appearing drug resistance of the given cell line determined in cell survival studies (Ex. 1, Fig. 1).

It has also been shown that the MAF value of a tumor cell line expressing a biologically inactive mutant MDR protein causing no drug resistance is zero indicating that the method according to the invention characterizes the MDR activity (i.e. the drug extruding potential) of the malignant tissues in a clinically relevant manner (Ex. 2).

As the method of the invention only makes use of several not unique characteristics of calcein-AM, it will be obvious for a person with ordinary skills in the art that any similar compound can be employed in the method of the invention instead of calcein-AM as far as all its important characteristics are similar to or identical with those of calcein-AM. Such characteristics of calcein-AM that are important with respect to the method of the invention are as follows: it is cell permeable; it is a good substrate of an MDR protein; and it is transformed in the cell by generally present enzymes to an easily detectable compound that is not a substrate (or not a good substrate) of the MDR protein and accumulates within the cell (its release from the cells is highly hindered).

According to one aspect of the present invention, a method is provided for the quantitative *in vitro* determination in biological specimens of the activity and/or concentration of a transport protein causing multi-drug resistance in tumors, comprising:
contacting said specimen or a part of said specimen with a derivative of an easily detectable compound, said derivative being a good substrate of said transport protein and being able to enter the cells of said specimen, and within the cells of said specimen said derivative is transformed to an easily detectable compound as the consequence of the action of an enzyme(s) present within said cells, said easily detectable compound not being a substrate (or not being a good substrate) of said transport protein and accumulates within said cells;
determining quantitatively the rate of accumulation (F) of said easily detectable compound within said cells; and then
contacting said assayed specimen or another part of said assayed specimen with said derivative of said easily detectable compound in the presence of a transport blocking agent; and
determining quantitatively the rate of accumulation (F*) of said easily detectable compound within said cells in the same manner as previously; characterized in
   (i) calculating the value of the dimensionless MAF characteristic of the measure of the activity of said transport protein present in said specimen based on the empirically established correlation MAF = (F*-F)/F*; and
   (ii) optionally deducing and expressing in desired diagnostic units the concentration of the biologically active MDR protein in said specimen and/or the MDR activity of said specimen, said deduction being made from the previously calculated MAF value applying an empirically established table and/or graph and/or other empirically established mathematical correlation.

According to a specific embodiment of the present invention, a method for the determination of the activity and/or the concentration of protein MDR1 is provided.

The method according to the invention is advantageously performed on a mammalian specimen and more advantageously it is performed on a human clinical specimen.

In another specific embodiment of the invention advantageously a calcein derivative and more advantageously a calcein-acetoxy-methylester is employed as said derivative of the easily detectable compound.

In another specific embodiment of the invention advantageously an inhibitor of said transport protein or an excess of a good substrate of said transport protein and more advantageously verapamil is applied as said transport blocking agent.

In the method of the invention the rate of the accumulation of said easily detectable compound is advantageously characterized by a fluorescence measurement. In one specific embodiment of the present invention said fluorescence measurement is done in a flow cytometer.

It is another aspect of the present invention to provide reagent kits also comprising instructions for carrying out the methods of the invention.

The reagent kits according to the invention advantageously comprise a calcein derivative and/or an inhibitor or a good substrate of a transport protein and more advantageously they comprise verapamil.

Below we give the short description of the attached drawings.

Fig. 1 is a graph showing the results of a MDR activity assay according to the invention performed on differently multi-drug resistant P-338 murine leukemia cell lines. In the assay shown in Fig. 1, cell suspensions of 2.5x10⁵/ml concentration were incubated in 0.25 µmol/l calcein-AM at 37 °C and the rate of fluorescence increase was followed by a fluorescent spectrophotometer. Elapsed time is shown on the abscissa and fluorescence intensity in arbitrary units is shown on the ordinate. It can be seen that the steepness of the starting slope of the curves decreases with increasing multi-drug resistance of the cell lines (order of multi-drug resistance is: P388-C < P388-MDRₐ < P388-MDR_{b}), while after addition of a transport blocking agent (verapamil) the steepness of the curves becomes similar for all the three cell lines. MAF values (0.06 < 0.37 < 0.87) calculated on the basis of the results shown in Fig. 1 proved to be in good correlation with the measure of multi-drug resistance of the respective cell lines determined in cytotoxicity experiments (see Ex. 1). Fig. 2 shows the quantitative determination of the activity of an MDR protein measured in a flow cytometer by detecting the accumulation of calcein. In the experiments shown in Fig. 2 a drug sensitive (control, cell line KB3) and three increasingly multi-drug resistant (cell lines KB-V1_{a-c}) human epidermoid carcinoma cell lines were examined. Cell suspensions of 2.5x10⁵/ml concentration were incubated for 10 min in 0.25 µmol/l calcein-AM at 37 °C and the fluorescence of the cells was measured in a flow cytometer. In parallel experiments, the accumulation of calcein was also measured on another parts of the same specimens that were pretreated with 100 µmol/l verapamil for 5 min (at 25 °C).

As it is well demonstrated on Fig. 2, the accumulation of calcein is decreasing with the increasing multi-drug resistance of the different cell lines (dark histograms). If MDR activity is previously blocked by incubation with verapamil, the measure of calcein accumulation reaches the control level (empty histograms). There is no detectable effect of the transport blocking agent verapamil on calcein accumulation of the control cell line (KB3). The logarithm of the intensity of green fluorescence is shown on the abscissa and cell count is shown on the ordinate. The rate of calcein accumulation and the MAF values can be calculated using the appropriate values of the shown hiss tograms (se Ex. 1).

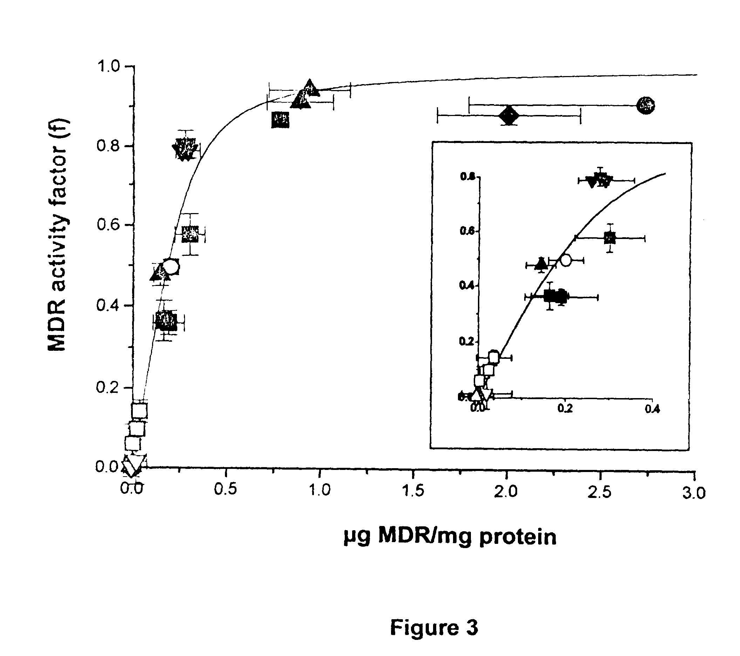

Fig. 3 is a graph showing the correlation between MAF and the concentration of the MDR protein determined by Western blot technique using the MDR-specific antibody 4077 [Tanaka, S. et al. Biochem. Biophys. Res. Commun. 166, 180 (1990)]. All cell lines used in the demonstrated experiments are, unless specific references are given, generally available commercial cell lines. Control cell lines: murine leukemia P388-C (□), human erythroid leukemia K562C (∇), human epidermoid carcinoma KB3 (Δ), murine fibroblast NIH3T3-C (O). Differently multi-drug resistant assayed cell lines: murine leukemia P388-MDR_{a-b} (■), human erythroid leukemia K562-MDR_{a-b} (▼), human epidermoid carcinoma KBV1_{a-c} (▲), murine erythroid leukemia F4-6 (◆) and NIH3T3-MDR, a murine fibroblast cell line stably transfected with human MDR1 (●) [Bruggemann, E. P. et al, J. Biol. Chem. 267, 21020 (1992)].

As it is shown on Fig. 3, the value of MAF is monotonously increasing with the increasing concentration of expressed MDR protein. In the clinically relevant range of concentration (0-0.2 µg MDR protein / mg total protein), the value of MAF is proportional to the protein concentration (see the magnified insert on Fig. 3). It can also be seen that with decreasing concentrations of the expressed protein the sensitivity of the method of the invention is increasing (since the slope of the curve becomes steeper).

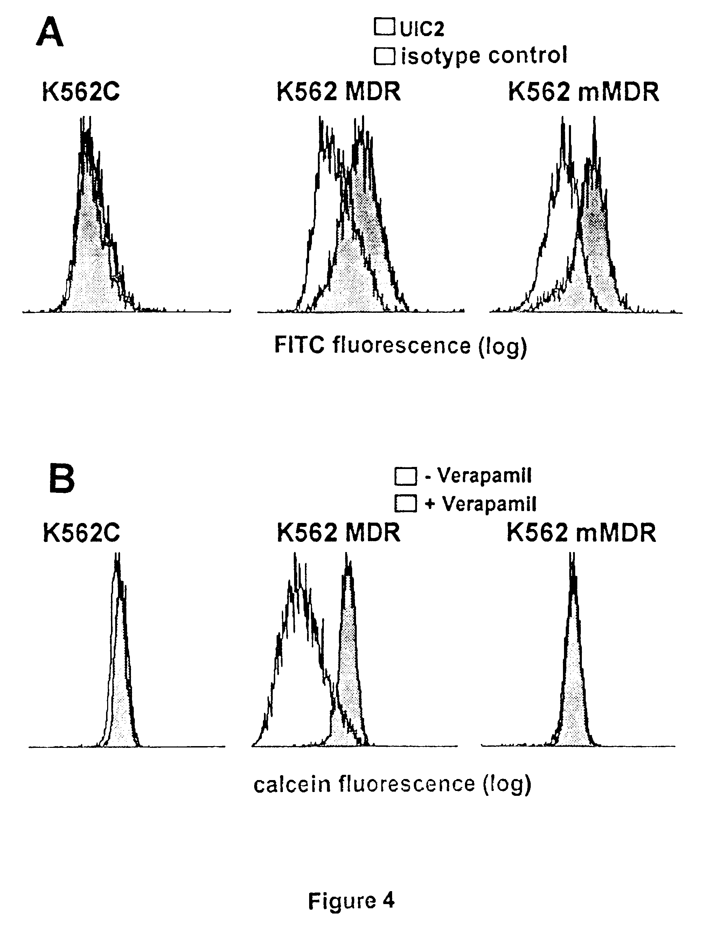

Fig. 4 depicts the results of an assay performed on three different human erythroid leukemia cell lines: K562C (control) - a drug sensitive cell line, K562-MDR - an MDR protein expressing cell line and K562-mMDR - a cell line expressing an inactive mutant MDR protein. Concentration of the expressed MDR protein was determined by immunofluorescence applying the human MDR1 specific antibody UIC2 [Mechetner, E. B. et al. Proc. Natl. Acad. Sci. USA 89, 5824 (1992)]. In the control experiment a murine IgG1 isotype antibody was used instead of the specific antibody. Signal was made visible using a FITC conjugated anti-murine IgG second antibody and the fluorescence of the cells was measured in a flow cytometer. The logarithm of the intensity of green fluorescence is shown on the abscissa and cell count is shown on the ordinate. As it can be seen on panel A, the level of MDR1 expression in cell line K562C is very low but the other two cell lines show significant MDR1 expression.

Panel B of Fig. 4 depicts the determination of MDR activity of the same cell lines by measuring the accumulation of calcein in a flow cytometer as shown on Fig. 2. Cell suspensions of 2.5x10⁵/ml concentration were incubated for 10 min in 0.25 µmol/l calcein-AM at 37 °C and the fluorescence of the cells was measured in a flow cytometer (empty histograms). In parallel experiments, the accumulation of calcein was also measured on another parts of the same specimens that were pretreated with 100 µmol/l verapamil for 5 min (at 25 °C, dark histograms). As it is shown on panel B of Fig. 4, the measured MDR activity is very low (f = 0.11) in the case of cell line K562C expressing low levels of the MDR protein. MDR activity of cell line K562-MDR expressing high levels of a functional MDR protein is high (f = 0.81), while in cell line K562-mMDR expressing an inactive mutant MDR protein, there is practically no MDR activity (f= 0.006).

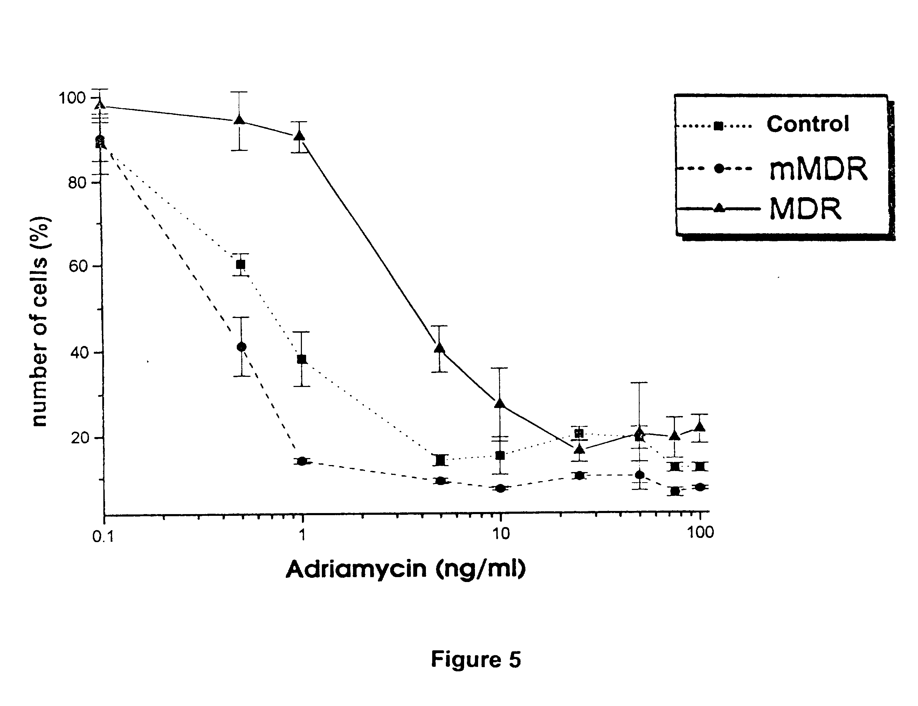

Fig. 5 depicts a cytotoxicity assay determining the drug resistance of the cell lines shown in Fig. 4. Cells were cultured for 3 days under standard conditions in the presence of 0.1-100 ng/ml adriamycin. The number of cells in the different cell cultures is given as the percentage of the number of cells counted in the culture not treated with the drug.

As it can well be seen on Fig. 5, the really expressed drug resistance is in much better accordance with the results obtained by the method of the invention based on the measurement of calcein accumulation than with those obtained by the traditional immunological measurement of MDR expression. Though cells expressing the inactive mutant MDR protein contain a high amount of said MDR protein, their multi-drug resistance is, in fact, in accordance with their real MDR activity that can be well characterized by MAF calculated on the basis of the measurement of calcein accumulation.

### Example 1.

### Quantitative in vitro determination of an MDR protein by measuring calcein accumulation (by fluorescent photometry and flow cytometry) in different tumor cell lines; correlation between the level of MDR protein expression and the measured MDR activity

In the experiments represented on Fig. 1, P-388 murine leukemia cell lines of different levels of multi-drug resistance were employed. In the cytotoxicity assay the adriamycin resistance of cell line P388-C (control cell line) proved to be IC₅₀ = 0.5 ng/ml, while the measured IC₅₀ values for P388-MDRₐ and P388-MDR_{b} were 4.0 ng/ml and 9.0 ng/ml, respectively. In the experiments shown in Fig. 1 cell suspensions of 2.5x10⁵/ml concentration were incubated in 0.25 µmol/l calcein-AM at 37 °C and the rate of fluorescence increase was followed by a fluorescent spectrophotometer. Elapsed time is shown on the abscissa and fluorescence intensity in arbitrary units is shown on the ordinate.

In the control cells (P388-C), there is a rapid accumulation of calcein and the rate of accumulation is not influenced by the addition of a transport blocking agent (100 µmol/l verapamil). In the multi-drug resistant cells (P388-MDR_{a-b}), however, the initial rate of calcein accumulation, without the addition of a transport blocking agent, is much lower but after adding a transport blocking agent this initial rate significantly increases. The method of the invention can similarly be applied in the concentration ranges of 10⁵-2x10⁶/ml cells and 0.05-1 µmol/l calcein-AM. To make the determination quantitative, it is necessary to reach a 100 % inhibition of the MDR activity so when applying a competitive inhibitor of the MDR protein present in the sample as a transport blocking agent (e.g. verapamil) it should be applied in high excess to the applied easily detectable compound derivative (e.g. calcein-AM). According to the method of the invention we have determined the accumulation rate of the easily detectable compound (e.g. calcein) without (F) and with the addition of a transport blocking agent (F*) from the slopes of the steady parts of the measured curves. The MAF values calculated using the MAF = (F*-F)/F* empirical correlation were as follows:

| | |
|---|---|
| P388-C | 0.059, |
| P388-MDRₐ | 0.365, |
| P388-MDR_{b} | 0.867. |

These values are in very good accordance with the drug resistance values determined in the cytotoxicity assays.

We have demonstrated on Fig. 2 the quantitative determination of the activity of an MDR protein measured in a flow cytometer by detecting the accumulation of calcein. In the experiments shown in Fig. 2 a drug sensitive (control, cell line KB3) and three increasingly multi-drug resistant (cell lines KB-V1_{a-c}) human epidermoid carcinoma cell lines were examined. Cell suspensions of 2.5x10⁵/ml concentration were incubated for 10 min in 0.25 µmol/l calcein-AM at 37 °C and the fluorescence of the cells was measured in a flow cytometer. In parallel experiments, the accumulation of calcein was also measured on another parts of the same specimens that were pretreated with 100 µmol/l verapamil for 5 min (at 25 °C).

As it is well demonstrated on Fig. 2, the accumulation of calcein is decreasing with the increasing multi-drug resistance of the different cell lines (dark histograms). If MDR activity is previously blocked by incubation with verapamil, the measure of calcein accumulation reaches the control level (empty histograms). There is no detectable effect of the transport blocking agent verapamil on calcein accumulation of the control cell line (KB3). The logarithm of the intensity of green fluorescence is shown on the abscissa and cell count is shown on the ordinate. As all the specimens were incubated for the same time (10 min) with calcein-AM, the measure of the accumulation of calcein detected exactly after 10 minutes can be taken as the rate of calcein accumulation. Thus MAF values calculated from the fluorescence values determined after exactly 10 min of incubation without (F) and with the addition of a transport blocking agent (F*), based on the empirically established correlation MAF = (F*-F)/F* were as follows:

| | |
|---|---|
| KB3 | 0.02, |
| KB-V1ₐ | 0.43, |
| KB-V1_{b} | 0.74, |
| KB-V1_{c} | 0.92. |

Instead of the absolute fluorescence values, the mean channel numbers in a logarithmic scale can also be used when calculating the MAF value. In such a case a modified empirical correlation is to be used for the calculation as follows:${\text{MAF = 1-e}}^{\text{k(G-G*)}}$ wherein G is the mean channel number measured in the absence of a transport blocking agent, G* is the mean channel number measured in the presence of a transport blocking agent and k is a constant used for the calculation of the absolute fluorescence values that is characteristic of the flow cytometer applied.

On Fig. 3 the correlation between MAF and the concentration of the MDR protein is shown. MAF values were determined for several differently multi-drug resistant cell lines by the fluorescent photometric method shown on Fig. 1 on the basis of the calcein accumulation curves.

In parallel experiments the concentration of the expressed MDR protein in the same cell lines were determined by Western blot technique applying the MDR-specific antibody 4077. For the description of the applied materials and methods see the following publications: Homolya, L. et al, J. Biol. Chem., 268, 21493 (1993) and Holló, Zs. et al, Biochim. Biophys. Acta 1191, 384 (1994). The concentration of the expressed MDR protein was determined from the measured luminescence values by comparison with a specimen of known MDR protein concentration. Control cell lines: murine leukemia P388-C (□), human erythroid leukemia K562C (∇), human epidermoid carcinoma KB3 (Δ), murine fibroblast NIH3T3-C (O). Differently multi-drug resistant assayed cell lines: murine leukemia P388-MDR_{a-b} (■), human erythroid leukemia K562-MDR_{a-b} (▼), human epidermoid carcinoma KBV1_{a-c} (▲), murine erythroid leukemia F4-6 (◆) and NIH3T3-MDR, a murine fibroblast cell line stably transfected with human MDR1 (●).

As it is shown on Fig. 3, the value of MAF is monotonously increasing with the increasing concentration of expressed MDR protein. In the clinically relevant range of concentration (0-0.2 µg MDR protein / mg total protein), the value of MAF is proportional to the protein concentration (see the magnified insert on Fig. 3). Additionally, it can also be seen that with decreasing concentrations of the expressed protein the sensitivity of the method of the invention is increasing (since the slope of the curve becomes steeper).

### Example 2

### Quantitative in vitro determination of an expressed mutant MDR protein by using an immunological method and the quantitative determination of its MDR activity by measuring calcein accumulation

On Fig. 4, we have demonstrated the results of an assay performed on three different human erythroid leukemia cell lines: K562C (control) - a drug sensitive cell line, K562-MDR - an MDR protein expressing cell line and K562-mMDR - a cell line expressing an inactive mutant MDR protein. Concentration of the expressed MDR protein was determined by immunofluorescence applying the human MDR1 specific antibody UIC2. In the control experiments a murine IgG1 isotype antibody was used instead of the specific antibody. Antibody labeling was visualized by using an FITC conjugated anti-murine IgG second antibody and the fluorescence of the cells was measured in a flow cytometer. The logarithm of the intensity of green fluorescence is shown on the abscissa and cell count is shown on the ordinate. As it can be seen on panel A, the level of MDR1 expression in cell line K562C is very low but the other two cell lines show significant MDR1 expression.

On panel B of Fig. 4, the determination of MDR activity of the same cell lines is shown by measuring the accumulation of calcein in a flow cytometer as shown on Fig. 2. Cell suspensions of 2.5x10⁵/ml concentration were incubated for 10 min in 0.25 µmol/l calcein-AM at 37 °C and the fluorescence of the cells was measured in a flow cytometer (empty histograms). In parallel experiments, the accumulation of calcein was also measured on another parts of the same specimens that were pretreated with 100 µmol/l verapamil for 5 min (at 25 °C, dark histograms). As it is shown on panel B of Fig. 4, the measured MDR activity is very low (f = 0.11) in the case of cell line K562C expressing low levels of the MDR protein. MDR activity of cell line K562-MDR expressing high levels of a functional MDR protein is high (f = 0.81), while in cell line K562-mMDR expressing an inactive mutant MDR protein, there is practically no MDR activity (f = 0.006).

On Fig. 5, we have demonstrated the results of a cytotoxicity assay determining the drug resistance of the cell lines shown in Fig. 4. Cells were cultured for 3 days under standard conditions in the presence of 0.1-100 ng/ml adriamycin. The number of cells in the different cell cultures is given as the percentage of the number of cells counted in the culture not treated with the drug.

The results of the three assay performed are summarized in the following table:

| Cell line | MDR concentration (UIC2 signal compared to the control) | MAF value | Resistance to Adriamycin (IC₅₀, ng/ml) |
|---|---|---|---|
| K562-C | 1.09 x | 0.11 | 0.7 |
| K562-MDR | 3.98 x | 0.81 | 3.1 |
| K562-mMDR | 4.18 x | 0.006 | 0.35 |

As it can well be seen from the above table, the really expressed drug resistance is in much better accordance with the results obtained by the method of the invention based on the measurement of calcein accumulation than with those obtained by the traditional immunological measurement of MDR expression. Though cells expressing the inactive mutant MDR protein contain a high amount of said MDR protein, their multi-drug resistance is, in fact, in accordance with their real MDR activity that can be well characterized by MAF calculated on the basis of the measurement of calcein accumulation.

From the experimental results demonstrated above and from the accompanying teachings, it should be evident for the skilled artisan that the assay methods according to the invention enable the quantitative functional *in vitro* clinical diagnostic determination of the activity and concentration of proteins causing multi-drug resistance in different tumors such as MDR1 or P-glycoprotein. This is why the method of the present invention, by the in *vitro* determination of the MDR activity expressed in the tumor cells, makes possible a better planing and execution of chemoterapeutic treatments in case of multi-drug resistant tumors reducing both the detrimental side effects and the costs of said treatments.

## Claims

1. A method for the quantitative *in vitro* determination in biological specimens of the activity and/or concentration of a transport protein causing multi-drug resistance in tumors (MDR protein), comprising
contacting said specimen or a part of said specimen with a derivative of an easily detectable compound, said derivative being a good substrate of said transport protein and being able to enter the cells of said specimen, and within the cells of said specimen being transformed to an easily detectable compound as the consequence of the action of enzyme(s) present within said cells; said easily detectable compound not being a substrate (or not being a good substrate) of said transport protein and therefore accumulating within said cells;
determining quantitatively the rate of accumulation (F) of said easily detectable compound within said cells; and then
contacting said assayed specimen or another part of said assayed specimen with said derivative of said easily detectable compound in the presence of a transport blocking agent; and
determining quantitatively the rate of accumulation (F*) of said easily detectable compound within said cells in the same manner as previously; characterized in
(i) calculating the value of the dimensionless MDR activity factor (MAF) that is illustrative of the measure of the activity of said transport protein present in said specimen using the empirically established correlation: MAF = (F*-F)/F*; and
(ii) optionally deducing, and expressing in desired diagnostic units, the concentration of the biologically active MDR protein in said specimen and/or the MDR activity of said specimen, said deduction being made from the previously calculated MAF value applying an empirically established table and/or graph and/or other empirically established mathematical correlation.

2. The method of claim 1, characterized in determining the activity and/or the concentration of transport protein MDR1.

3. The method of claim 1 or 2, characterized in determining the activity and/or the concentration of said transport protein in a mammalian specimen.

4. The method of claim 3, characterized in determining the activity and/or the concentration of said transport protein in a human clinical specimen.

5. A reagent kit, also comprising instructions, for the quantitative *in vitro* detection in biological specimens of the activity and/or concentration of a transport protein causing multi-drug resistance in tumors (MDR protein) comprising;
a) a derivative of an easily detectable compound, said derivative being a good substrate of said transport protein and being able to enter the cells of said specimen, and within the cells of said specimen being transformed to an easily detectable compound as the consequence of the action of enzyme(s) present within said cells; said easily detectable compound not being a substrate (or not being a good substrate) of said transport protein and therefore accumulating within said cells; and
b) a transport blocking agent.

6. The method or kit of any one of claims 1-5, wherein said derivative of said easily detectable compound is a calcein derivative.

7. The method or kit of claim 6, wherein said calcein derivative is calcein-acetoxy-methylester.

8. The method or kit of claims 1-7, wherein said transport blocking agent is an inhibitor of said transport protein.

9. The method or kit of any one of claims 1-7, wherein said transport blocking agent is an excess of a good substrate of said transport protein.

10. The method or kit of claim 9, wherein said good substrate is verapamil.

11. The method of any one of claims 1-10, wherein the rate of accumulation of said easily detectable compound within said cells is characterized by a fluorescence measurement.

12. The method of claim 11, wherein said fluorescence measurement is done in a flow cytometer.

## Patentansprüche

1. Verfahren zur quantitativen *in vitro* Bestimmung der Aktivität und/oder der Konzentration eines in Tumoren Multidrogen-Resistenz verursachenden Transportproteins (MDR-Proteins) in biologischen Proben,
wobei die Proben oder ein Teil von ihnen mit dem Derivat einer leicht detektierbaren Substanz kontaktiert werden, das für das erwähnte Transportprotein ein gutes Substrat ist, fähig ist, in die Zellen der Probe einzudringen und innerhalb der Zellen durch in diesen befindliche Enzyme zu einer leicht detektierbaren Verbindung umgesetzt zu werden, die für das erwähnte Transportprotein kein Substrat (oder kein gutes Substrat) ist und sich deshalb in den Zellen anreichert,
dann die Geschwindigkeit (F) der Anreicherung der erwähnten leicht detektierbaren Verbindung in den Zellen quantitativ gemessen wird,
die untersuchte Probe oder ein anderer Teil von ihr mit dem Derivat der erwähnten leicht detektierbaren Verbindung in Gegenwart eines Transportblockers kontaktiert wird und
die Geschwindigkeit (F*) der Anreicherung der erwähnten leicht detektierbaren Verbindung in den Zellen auf die gleiche Weise wie zuvor quantitativ gemessen wird, *dadurch gekennzeichnet,* daß
(i) auf Grund des empirisch erhärteten Zusammenhanges MAF = (F*-F)/F* der Wert des für das Maß der Aktivität des erwähnten, in der Probe befindlichen Transportproteins charakteristischen dimensionslosen MDR-Aktivitätsfaktors (MAF) berechnet wird und
ii) gegebenenfalls die Konzentration des biologisch aktiven MDR-Proteins in den Proben und/oder die MDR-Aktivität der Proben aus dem vorher berechneten MAF-Wert mit Hilfe einer empirisch aufgestellten Tabelle, einer empirisch aufgestellten Kurve und/oder sonstiger empirisch erhärteter mathematischer Zusammenhänge deduktiv bestimmt und in den gewünschten diagnostischen Einheiten ausgedrückt wird.

2. Verfahren nach Anspruch 1, dadurch gekennzeichnet, daß Aktivität und/oder Konzentration des Transportproteins MDR1 bestimmt werden.

3. Verfahren nach Anspruch 1 oder 2, dadurch gekennzeichnet, Aktivität und/oder Konzentration des genannten Transportproteins in aus Säugern stammenden biologischen Proben untersucht werden.

4. Verfahren nach Anspruch 3, dadurch gekennzeichnet, daß die Aktivität und/oder Konzentration des genannten Transportproteins in klinischen Humanproben untersucht werden.

5. Reagenz-Kit, der auch Instruktionen enthält, zur quantitativen *in vitro* Bestimmung der Aktivität und/oder der Konzentration eines in Tumoren Multidrogen-Resistenz verursachenden Transportproteins (MDR-Proteins) in biologischen Proben, enthaltend
a) ein Derivat einer leicht detektierbaren Verbindung, das ein gutes Substrat für das genannte Transportprotein ist, fähig ist, in die Zellen der Probe einzudringen und innerhalb der Zellen durch in diesen befindliche Enzyme zu einer leicht detektierbaren Verbindung umgesetzt zu werden, die für das erwähnte Transportprotein kein Substrat (oder kein gutes Substrat) ist und sich deshalb in den Zellen anreichert, und
b) einen Transportblocker.

6. Verfahren oder Kit nach einem der Ansprüche 1-5, in dem das Derivat der leicht detektierbaren Verbindung ein Calcein-Derivat ist.

7. Verfahren oder Kit nach Anspruch 6, in dem das Calcein-Derivat Calcein-acetoxy-methylester ist.

8. Verfahren oder Kit nach einem der Ansprüche 1-7, in dem der erwähnte Transportblocker ein Inhibitor des Transportproteins ist.

9. Verfahren oder Kit nach einem der Ansprüche 1-7, in dem der erwähnte Transportblocker ein Überschuß eines guten Substrates des Transportproteins ist.

10. Verfahren oder Kit nach Anspruch 9, in dem das gute Substrat Verapamil ist.

11. Verfahren nach einem der Ansprüche 1-10, in dem die Geschwindigkeit der Anreicherung der leicht detektierbaren Verbindung in den Zellen durch Fluorescenzmessung charakterisiert wird.

12. Verfahren nach Anspruch 11, in dem die Fluorescenzmessung in einem Strömungscytometer vorgenommen wird.

## Revendications

1. Procédé pour la détermination quantitative in vitro dans un échantillon biologique de l'activité et/ou de la concentration d'une protéine transporteuse causant une résistance multi-drogue dans les tumeurs (protéine MDR),
au cours duquel
on contacte l'échantillon biologique ou une partie de l'échantillon avec un dérivé d'un composé facilement détectable, ce dérivé étant un bon substrat de la protéine transporteuse susmentionnée et étant capable d'entrer dans les cellules de l'échantillon biologique et étant transformé à l'intérieur des cellules de l'échantillon en un composé facilement détectable par la suite de l'action de l'un ou plusieurs enzymes présents à l'intérieur des cellules mentionnées; le composé facilement détectable n'étant pas un substrat (ou n'étant pas un bon substrat) de la protéine transporteuse, s'accumule à l'intérieur des cellules;
on détermine d'une manière quantitative la vitesse d'accumulation (F) du composé facilement détectable à l'intérieur des cellules; et ensuite
on contacte l'échantillon biologique testé ou une autre partie de cet échantillon avec le dérivé du composé facilement détectable en présence d'un agent inhibiteur du transport; et
on détermine d'une manière quantitative la vitesse d'accumulation (F*) du composé facilement détectable à l'intérieur des cellules comme précédemment;
caractérisé en ce qu'il consiste à
(i) calculer la valeur du facteur d'activité MDR sans dimension (MAF) qui est caractéristique au mesure de l'activité de la protéine transporteuse présente dans l'échantillon biologique, en utilisant la corrélation suivante établie empiriquement: MAF = (F*-F)/F*; et
(ii) le cas échéant déduire, et exprimer en l'unité diagnostique désirée, la concentration dans l'échantillon biologique de la protéine MDR ayant une activité biologique et/ou l'activité MDR de l'échantillon, la déduction étant faite de la valeur MAF calculée préalablement, en utilisant un tableau et/ou un diagramme et/ou une autre corrélation mathématique établis empiriquement.

2. Le procédé selon la revendication 1 caractérisé en ce qu'il consiste à déterminer l'activité et/ou la concentration de la protéine transporteuse MDR1.

3. Le procédé selon la revendication 1 ou 2, caractérisé en ce qu'il consiste à déterminer l'activité et/ou la concentration de la protéine transporteuse dans un échantillon biologique d'origine mammifère.

4. Le procédé selon la revendication 3, caractérisé en ce qu'il consiste à déterminer l'activité et/ou la concentration de la protéine transporteuse dans un échantillon biologique clinique humain.

5. Dispositif de réactifs ou "kit" comprenant d'instructions également pour la détection quantitative in vitro de l'activité et/ou de la concentration d'une protéine transporteuse causant une résistence multi-drogue dans les tumeurs (protéine MDR) dans un échantillon biologique comprenant:
a) un dérivé d'un composé facilement détectable, ce dérivé étant un bon substrat de la protéine transporteuse susmentionnée et étant capable d'entrer dans les cellules de l'échantillon biologique et étant transformé dans les cellules de l'échantillon en un composé facilement détectable par la suite de l'action de l'un ou plusieurs enzymes présents à l'intérieur des cellules mentionnées; le composé facilement détectable n'étant pas un substrat (ou n'étant pas un bon substrat) de la protéine transporteuse s'accumule à l'intérieur des cellules; et
b) un agent inhibiteur du transport.

6. Procédé ou kit selon l'une quelconque des revendications 1 à 5, dans lequel le dérivé du composé facilement détectable est un dérivé du calcein.

7. Procédé ou kit selon la revendication 6, dans lequel le dérivé du calcein est l'ester acetoxyméthylique du calcein.

8. Procédé ou kit selon l'une quelconque des revendications 1 à 7, dans lequel l'agent inhibiteur du transport est un inhibiteur de la protéine transporteuse.

9. Procédé ou kit selon selon l'une quelconque des revendications 1 à 7, dans lequel l'agent inhibiteur du transport est un bon substrat en excès de la protéine transporteuse.

10. Procédé ou kit selon selon la revendication 9, dans lequel le bon substrat est le vérapamil.

11. Procédé selon l'une quelconque des revendications 1 à 10, dans lequel la vitesse d'accumulation du composé facilement détectable à l'intérieur des cellules est caractérisée par un mesurement à fluorescence.

12. Le procédé selon la revendication 11, dans lequel le mesurement à fluorescence est fait par un cytometer à circulation.
